# EUROPEAN PATENT SPECIFICATION

(11) **EP 3 337 571 B1**
(45) Date of publication and mention of the grant of the patent: **30.06.2021**
(21) Application number: 15771368.6
(22) Date of filing: 14.09.2015
(51) Int. Cl.: A61K 8/81, A61K 8/892, A61Q 11/00, A61K 8/22, A61Q 17/00, A61K 8/891, A61K 8/894, A61K 8/31

(54) **ANHYDROUS TOOTH WHITENING COMPOSITIONS COMPRISING CETYLPYRIDINIUM CHLORIDE**
WASSERFREIES ZAHN-BLEICHMITTEL ENTHALTEND CETYLPYRIDINIUM CHLORID
COMPOSITION ANHYDRE DE BLANCHISSEMENT DES DENTS COMPRENANT DU CHLORURE DE CETYLPYRIDINIUM

(43) Date of publication of application: 27.06.2018
(73) Proprietor: Colgate-Palmolive Company, New York, NY 10022 (US)
(72) Inventor: ROBBINS, Kyle, Toms River, New Jersey 08753 (US); PILLAI, Shyamala, Piscataway, NJ 08854 (US); NESTA, Jason, Piscataway, NJ 08854 (US); SMITH-WEBSTER, Kimdra, deceased (US)
(74) Representative: Sonnenhauser, Thomas Martin
(86) International application number: PCT/US2015/049884
(87) International publication number: WO 2017/048216

(56) References cited:
- WO-A1-2011/112193
- WO-A2-2005/018593
- US-A1- 2014 308 322
- None

## Description

### BACKGROUND

Disclosed herein are anhydrous compositions comprising cetylpyridinium chloride, such as oral care compositions. Oral care compositions disclosed herein include tooth whitening compositions. Further disclosed herein are methods of using anhydrous compositions comprising cetylpyridinium chloride to reduce microbial growth due to consumer use, for example microbial growth on external surfaces of oral care devices, such as toothbrushes and applicators.

There is a high consumer demand for tooth whitening products. It has become desirable for a person's teeth to appear bright or "white." Society places a high value on the "whiteness" of one's teeth. One whose teeth are white may enjoy more personal confidence and satisfaction and may even enjoy greater social acceptance. Many substances that a person confronts or comes in contact with on a daily basis, however, can stain or reduce the whiteness of one's teeth. In particular, the foods, tobacco products, and fluids that one consumes tend to stain one's teeth. These products or substances tend to accumulate on the enamel layer of the tooth and form a pellicle film over the teeth.

These staining and discoloring substances can then permeate the enamel layer. This problem occurs gradually over many years, but imparts a noticeable discoloration of the enamel of one's teeth. So long as the discolored teeth are still healthy and do not pose any health risk or problem, a product or substance that would whiten the discolored teeth would be advantageous. Tooth whitening products that are to be used at home or in private by the consumer should also be safe and easy to use and be stable and retain their whitening efficacy during storage on retail store shelves as well as over the period of use by the consumer, without degradation or microbial build-up over the product's lifetime.

Products and substances that are presently available to whiten teeth may include a variety of different ingredients, but the primary active ingredient is a peroxide agent formulated into a liquid, paste or gel carrier. Peroxide retention on the tooth surface may be achieved by incorporating the peroxide agent into an adhesive, for example a pressure sensitive adhesive, that can adhere to the tooth surface, in addition to at least one adhesion enhancing agent.

As the consumer applies a tooth whitening composition to his teeth with an oral care device, such as an applicator, it is inevitable that a certain amount of saliva, containing bacteria and other microbes from the mouth, will contact the surface of the device as the consumer uses it, and therefore be transferred to the oral care device. This bacteria may multiply and, over time, result in microbial build-up and a resultant odor on the applicator that is unsatisfactory to the consumer.

There is thus a need in the art for improved tooth whitening compositions that can provide good anti-microbial efficacy on the applicator device during storage and use by the consumer.

Cetylpyridinium chloride (CPC) is a cationic quaternary ammonium compound that has previously been shown to have antimicrobial efficacy when dispersed in an aqueous composition. CPC is a common preservative used in mouthwash and other aqueous compositions. However, CPC has not previously been incorporated into non-aqueous whitening compositions, such as tooth whitening compositions, as it is highly water soluble.

Disclosed herein is an anhydrous tooth whitening composition comprising cetylpyridinium chloride and methods of using such anhydrous tooth whitening compositions comprising cetylpyridinium chloride to reduce microbial growth and the malodors associated with microbial growth.

### BRIEF SUMMARY

Disclosed herein are anhydrous tooth whitening compositions comprising at least one hydrophobic polymer carrier, cetylpyridinium chloride, and a cross-linked PVP-H₂O₂ complex whitening agent, wherein the composition is free of water.

In certain embodiments, hydrophobic polymer carrier is a silicone pressure sensitive adhesive. The cross-linked PVP-H₂O₂ complex may comprise from 0.1% to 6.0% by weight hydrogen peroxide, relative to the total weight of the composition.

In various embodiments of the disclosure, the cetylpyridinium chloride is present in the composition in an amount ranging from about 0.05% to about 0.20% by weight relative to the total weight of the composition, such as, for example, about 0.1% by weight relative to the total weight of the composition.

The anhydrous tooth whitening composition disclosed herein may, in certain embodiments, comprise at least two adhesion enhancing agents. In certain exemplary embodiments, the at least two adhesion enhancing agents may be chosen from fumed silica and petrolatum. In certain embodiments, the anhydrous tooth whitening composition disclosed herein may be free of polyethylene, and in certain embodiments, the anhydrous tooth whitening composition may be in the form of a gel.

Further disclosed herein are methods for reducing microbial growth on the surface of an oral care device comprising contacting the oral care device with saliva and with an anhydrous tooth whitening composition, wherein the tooth whitening composition comprises at least one hydrophobic polymer carrier, cetylpyridinium chloride, and a cross-linked PVP-H₂O₂ complex whitening agent, wherein the composition is free of water.

In certain embodiments of the methods for reducing microbial growth disclosed herein, after the contacting step, the oral care device is free of the malodor caused by bacterial growth for a time period of at least about one month, such as at least about two months or at least about three months. In certain embodiments, the cross-linked PVP-H₂O₂ complex comprises from 0.1% to 6.0% by weight hydrogen peroxide, relative to the total weight of the composition.

According to various exemplary methods for reducing microbial growth disclosed herein, the cetylpyridinium chloride is present in the composition in an amount ranging from about 0.05% to about 0.20% by weight relative to the total weight of the composition, such as about 0.1%) by weight relative to the total weight of the composition.

In certain embodiments of the methods for reducing microbial growth disclosed herein, the anhydrous tooth whitening composition further comprises at least two adhesion enhancing agents. In certain exemplary embodiments, the at least two adhesion enhancing agents may be chosen from fumed silica and petrolatum. In certain embodiments of the methods disclosed herein, the anhydrous tooth whitening composition may be free of polyethylene, and in certain embodiments, the anhydrous tooth whitening composition may be in the form of a gel.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiments is merely exemplary in nature and is in no way intended to limit the invention, its application, or uses.

As used throughout, ranges are used as shorthand for describing each and every value that is within the range. Any value within the range can be selected as the terminus of the range. In addition, all references cited herein are hereby incorporated by reference in their entireties. In the event of a conflict in a definition in the present disclosure and that of a cited reference, the present disclosure controls.

As used herein, the term "one or more of ' with respect to a listing of items such as, for example, A and B, means A alone, B alone, or A and B. The term "at least one of' is used to mean one or more of the listed items can be selected.

Unless otherwise specified, all percentages and amounts expressed herein and elsewhere in the specification should be understood to refer to percentages by weight. The amounts given are based on the active weight of the material.

There is a need in the art for improved tooth whitening compositions that can provide whitening efficacy and incorporate ingredients that are appealing to consumers, while providing a stable formulation that inhibits microbial growth and therefore does not result in an unpleasant odor over time as the consumer uses and stores the tooth whitening composition. Accordingly, disclosed herein are tooth whitening compositions comprising at least one hydrophobic polymer carrier, cetylpyridinium chloride, and a cross-linked PVP-H₂O₂ complex whitening agent, wherein the composition is anhydrous or free of water.

The tooth whitening compositions disclosed herein may further comprise other additional ingredients that include those known to one of skill in the art, including one or more of the following components: fluoride ion sources, surfactants, flavoring agents, sweeteners, desensitizing agents, additional antimicrobial agents, anti-caries agents, anti-calculus agents, tartar control agents, anti-inflammatory agents, vitamins, pigments and coloring agents, preservatives, and enzymes, as will be discussed in greater detail below.

In some embodiments, the tooth whitening compositions disclosed herein are viscous fluids, such as gels, which maintain their consistency during storage, thereby enabling the product to be applied to the tooth surface, such as with a soft applicator pen or brush.

In certain embodiments, the tooth whitening composition disclosed herein is a gel. The viscosity of the tooth whitening composition may be greater than about 1,000 centipoise (cPs) and less than about 900,000 cPs, such as ranging from about 10,000 cPs to about 100,000 cPs; from about 50,000 to about 900,000 cPs, or ranging from about 200,000 cPs to about 600,000 cPs.

### Hydrophobic Polymer Carriers

The at least one hydrophobic polymer carrier of the tooth whitening compositions disclosed herein, may, in certain embodiments, be insoluble in water and stable for longer durations during exposure to saliva and other aqueous solutions found in an oral cavity, as compared to prior art water-soluble whitening solutions.

The term "hydrophobic" or "water-insoluble" as applied to polymers and as employed herein refers to polymers which are substantially non-aqueous having a water solubility of less than one gram per 100 grams of water at 25 °C. In various embodiments, a hydrophobic polymer is compatible with the at least one whitening agent described herein. In certain embodiments, a hydrophobic polymer is selected for the carrier to produce a tooth whitening composition having a viscosity ranging from about 1,000 cPs to about 900,000 cPs, such as from about 10,000 cPs to about 900,000 cPs or from about 10,000 cPs to about 100,000 cPs.

One class of hydrophobic polymers that may be used according to certain exemplary embodiments comprise siloxane polymers, which are also generally known in the art as "silicone" polymers, such as silicone pressure sensitive adhesives (PSA). In certain embodiments disclosed herein, the hydrophobic polymers in the carrier are those in which a whitening agent can be dispersed and are well known in the art. Many such silicone polymers are commercially available. In various embodiments, a silicone-based hydrophobic polymer is a polyorganosiloxane, such as polydimethylsiloxane.

Exemplary polyorganosiloxanes may be produced by condensing a silicone resin and an organosiloxane, such as a polydiorganosiloxane. Such hydrophobic polymers are an elastomeric, tacky material, adhesion of which to dental enamel surfaces can be varied by altering the ratio of silicone resin to polydiorganosiloxane in the copolymer molecule. In certain embodiments, the polymers are pressure sensitive hydrophobic polymers specifically designed for pharmaceutical use and are permeable to many drug compounds and find application for the transdermal application of various compounds. In one such embodiment, the silicone polymers are the copolymer product of mixing a silanol terminated polydiorganosiloxane, such as polydimethyl siloxane, with a silanol-containing silicone resin whereby the silanol groups of the polydiorganosiloxane undergo a condensation reaction with the silanol groups of the silicone resin so that the polydiorganosiloxane is lightly crosslinked by the silicone resin (that is, the polydiorganosiloxane chains are bonded together through the resin molecules to give chain branching and entanglement and/or a small amount of network character) to form the silicone hydrophobic polymers. A catalyst, for example, an alkaline material, such as ammonia, ammonium hydroxide or ammonium carbonate, can be mixed with the silanol-terminated polydiorganosiloxane and the silicone resin to promote this crosslinking reaction. By copolymerizing the silicone resin with the silanol terminated polydiorganosiloxane, there results a polymer with self-adhering properties and the cohesive properties of a soft elastomer matrix characteristic of pressure sensitive polymers being distinguished from the hard, non-elastomeric properties of other silicone resins. In one embodiment, hydrophobic polymers used in the carrier are available from the Dow-Corning Company under the brand name BIO-PSA.

The modification of a ratio of silicone resin to polydiorganosiloxane modifies the tackiness of the hydrophilic polymer. This ratio may, for example, be in the range of 70:30 to 50:50. For example, the BIO-PSA silicone sold by Dow-Corning is available in three silicone resin to silicone polymer ratios: namely, 65/35 (low tack), 60/40 (medium tack), and 55/45 (high tack), as modifying the silicone resin to polydiorganosiloxane ratio of the PSA will modify the tackiness of the PSA. Such a polyorganosiloxane pressure sensitive adhesive is available dissolved in either ethyl acetate solvent or dimethicone. An exemplary suitable silicone PSA is Silicone Adhesive 8-7016, commercially available from Dow Corning.

In some embodiments, the siloxane polymers that can function as part of the hydrophobic component are in the form of a fluid. Polysiloxane fluids useful herein for the hydrophobic silicone material component include those with a viscosity, at 25° C, of about 1 milliPascal-sec (mPa-s) to about 1000 mPa-s, or about 2 mPa-s to about 500 mPa-s, or about 20 mPa-s to about 400 mPa-s. Polysiloxane fluids for use herein can be linear or cyclic, and can be substituted with a wide variety of substituents. In certain embodiments, substituents include methyl, ethyl and phenyl substituents. Suitable polysiloxane fluids include linear polysiloxane polymers such as dimethicone and other low viscosity analogues of the polysiloxane materials, in certain embodiments having a viscosity, at 25° C, of 200 mPa-s or less and cyclomethicone, and other cyclic siloxanes having for example a viscosity, at 25° C, of 200 mPa-s or less. Other fluids include polysiloxane polyether copolymers and hydroxy terminated polydimethyl-siloxane fluid (e.g., Dow Corning ST-DIMETHICONOL™ 40, Dow Corning SGM 36, SGM3). Commercial examples of materials that are suitable for use herein include the DC200 series fluids marketed by Dow-Corning Corporation and the AK Fluid series marketed by Wacker-Chemie GmbH, Munchen, Germany. High molecular silicone resins with a polysiloxane blend may also be used, including powdered trimethylsiloxysilicate, for example, Dow Corning 593 fluid, Wacker Belsil TMS 803. Another suitable silicone fluid from Dow Corning is Q7-9210.

In certain embodiments disclosed herein, the at least one hydrophobic polymer carrier, such as the polydiorganosiloxane, may comprises from about 10% to about 80% by weight of the tooth whitening composition, such as, for example about 20% to about 60% or from about 40% to about 60%.

### Cationic Quaternary Ammonium Compounds

In addition to the at least one hydrophobic polymer carrier, the tooth whitening compositions disclosed herein further comprises least the cationic quaternary ammonium compound cetylpyridinium chloride (CPC). Other exemplary cationic quaternary ammonium compounds that may be used in addition to CPC include lauryl trimethylammonium chloride, cetyl trimethylammonium bromide, di-isobutylphenoxyethyldimethylbenzylammonium chloride, coconut alkyltrimethylammonium nitrite, cetyl pyridinium fluoride, and mixtures thereof.

In certain embodiments, the tooth whitening compositions disclosed herein comprise at least one cationic quaternary ammonium compound, which may be present in an amount effective to reduce microbial growth and/or an amount effective to reduce the malodor associated with microbial growth.

CPC has previously been shown to have antimicrobial activity and is frequently used in addition to or in place of ethanol as an antiseptic and preservative in mouthwashes, toothpastes, lozenges, throat sprays, breath sprays, and nasal sprays. At effective concentrations, CPC kills bacteria and other microorganisms and has been shown to be effective in preventing dental plaque and reducing gingivitis. It is believed to kill bacteria by binding to the negatively charged phosphates of the bacterial cell membrane. CPC, however, is highly soluble in water, and therefore is typically incorporated in aqueous solutions to maximize its bioavailability. It has been discovered, however, that CPC may be incorporated into the anhydrous tooth whitening compositions disclosed herein and still effectively reduce microbial growth.

In various embodiments, the tooth whitening compositions disclosed herein may comprise an anti-microbial effective amount of CPC, wherein the tooth whitening composition is anhydrous, meaning it is free of water. As used herein the terms "anhydrous" and "free of water" are used interchangeably and mean about 0% by weight of water or an amount of water that is so low as to not have a reasonable chemical effect on the composition. The tooth whitening composition may comprise trace levels of water from ingredients or from product manufacture; however, such trace levels are insubstantial and do not interfere with the anhydrous character of the tooth whitening composition. Nonetheless, the CPC retains antimicrobial effectiveness while incorporated in the anhydrous tooth whitening compositions disclosed herein.

Accordingly, the anhydrous tooth whitening compositions disclosed herein may be exposed to the surface of an oral care device, such as a toothbrush or an applicator, for a period of time without resulting in the malodor typical of microbial growth. For example, in certain embodiments, the anhydrous tooth whitening compositions disclosed herein may be exposed to the surface of an oral care device for at least about one month, such as at least about two months or at least about three months, without resulting in the malodor typical of microbial growth.

In certain embodiments disclosed herein, the at least one cationic quaternary ammonium compound CPC may comprise less than or equal to about 10% by weight relative to the total weight of the tooth whitening composition, such as, for example, an amount ranging from about 0.05% to about 0.20%, or about 0.1% by weight.

### Adhesion Enhancing Agents

In various embodiments, the tooth whitening compositions disclosed herein comprise at least one, such as at least two, adhesion enhancing agents that act as structuring agents in the compositions and augment adhesion of the whitening composition to the surface of the tooth, i.e., adhesion to the enamel. In embodiments disclosed herein wherein there are at least two adhesion enhancing agents, the at least two adhesion enhancing agents may comprise a combination of fumed silica and petrolatum. The combination of the fumed silica and petrolatum act as structuring agents, creating a viscoelastic structure together with the hydrophobic polymer carrier, such that G'/G" is greater than or equal to 1. This viscoelastic structure ensures that the solid ingredients remain homogenously suspended in the tooth whitening composition, such that the composition remains stable and has an adequate shelf life after formulation.

As used herein, G' and G" may be measured by the use of a strain sweep test. A strain sweep test is a test may be used to obtain the rheology profile of viscoelastic fluid compositions, such as the anhydrous tooth whitening compositions disclosed herein. The strain sweep test indicates whether a composition is structured or not. Taking the ratio of the G' value to the G" value within a linear viscoelastic region gives what is known as the "Structural Parameter." In some embodiments, the tooth whitening compositions disclosed herein have a G' to G" ratio of greater than or equal to about 1, such as greater than or equal to about 1.5, greater than or equal to about 1.75, or greater than or equal to about 2.0.

In a strain sweep test, the amplitude of the applied strain varies in the range 0.1%<y<100% while the frequency of oscillations is kept constant. The viscoelastic response of the material to the applied oscillatory strain is measured in terms of G' and G", the viscous and loss moduli, and valuable information may be obtained this way. In general, G' represents energy storage within the viscoelastic structure, and G" represents dissipation of this energy through flow. The linear viscoelastic region (LVR) is determined by the region of the strain sweep in which G' and G" remain constant with respect to the applied strain, and the ratio of elastic to viscous contribution (G'/G") can be calculated based on the G' and G" values within the LVR. This ratio provides a good indication of how structured a composition is, with a higher G'/G" ratio indicating that a more robust structure is present within the system. The yield stress value may also be determined from a strain sweep experiment, by plotting the elastic stress (G' x Strain) vs. Strain. With this information at hand, one can determine whether a certain viscoelastic material exhibits more solid-like or more fluid-like properties, and in this particular case the data can be utilized effectively to determine whether various aesthetics and solid materials can be successfully suspended within the composition.

Fumed silica, also known as pyrogenic silica (CAS number 112945-52-5), is an inorganic material comprising amorphous silica. The individual silica particles may agglomerate into a three-dimensional structure having a low bulk density and high surface area. Exemplary fumed silicas may include CAB-O-SIL fumed silica manufactured by Cabot Corporation and AEROSIL fumed silica manufactured by Evonik Industries. In certain embodiments disclosed herein, the fumed silica may be present in the tooth whitening composition in an amount ranging from about 1% to about 5%, such as about 1.5% to about 3% or about 1% to about 2%, by weight relative to the total weight of the composition.

Petrolatum is a known viscosity modifier for use in various compositions. In certain embodiments disclosed herein, the petrolatum may be present in the tooth whitening composition in an amount ranging from about 1% to about 50%, such as from about 25% to about 35%, by weight relative to the total weight of the composition. In certain embodiments disclosed herein, the petrolatum may be present in the tooth whitening composition in an amount of about 35% or in an amount of about 25%, by weight relative to the total weight of the composition.

Additional adhesion enhancing agents may be used in accordance with various embodiments of the tooth whitening composition disclosed herein. Exemplary adhesion enhancing agents disclosed herein include inorganic materials as well as organic natural and synthetic polymers. In certain exemplary embodiments, the inorganic adhesion enhancing material, such as silica, may be surface treated to compatibilize the adhesion enhancing agent with the hydrophobic components in the tooth whitening composition.

Organic materials which may be included in the tooth whitening compositions disclosed herein to enhance the properties of the hydrophobic polymers may include adhesion enhancing agents such as waxes, inclusive of beeswax, mineral oil, plastigel (a blend of mineral oil and polyethylene), sorbitan sebacate behenate polymer, white petrolatum, shellac, versagel (blend of liquid paraffin, butene/ethylene/styrene hydrogenated copolymer), polyethylene waxes, microcrystalline waxes, polyisobutene, polyvinylpyrrolidone/vinyl acetate copolymers, and insoluble polyacrylate copolymers. One adhesion enhancing agent that may be mentioned is a plastigel sold under the name PLASTIGEL 5, which is a blend of 5% polyethylene in mineral oil, available from Lyne Laboratories, Brockton, Mass. USA. The plastigel may be present in the composition in an amount ranging from about 20% to about 70% by weight, such as from about 30%) to about 60% by weight, based on the weight of the composition.

In certain exemplary embodiments, the tooth whitening compositions disclosed herein may be free of polyethylene. As used herein, the term "free of polyethylene" means about 0% by weight of polyethylene or an amount of polyethylene that is so low as to not have a reasonable chemical effect on the composition.

The tooth whitening compositions disclosed herein may further comprise crospovidone (poly[N-vinyl-2-pyrrolidone]) as an adhesion enhancing agent. Crospovidone may be present in the composition in an amount ranging from about 10% to about 30%, by weight relative to the total weight of the tooth whitening composition, such as ranging from about 15% to about 25%, or ranging from about 18% to about 25%.

Also effective as adhesion enhancing agents are liquid hydrophilic polymers including polyethylene glycols, nonionic polymers of ethylene oxide having the general formula: HOCH₂(CH₂OCH₂)ₙCH₂OH, wherein n represents the average number of oxyethylene groups. Polyethylene glycols available from Dow Chemical are designated by a number such as 200, 300, 400, 600, and 2000, which represents the approximate average molecular weight of the polymer, as well as nonionic block copolymer of ethylene oxide and propylene oxide of the formulas: HO(CH₄O)ₐ(C₃H₆O)_{b}(C₂H_{4O})_{c}H.

The block copolymer may be chosen (with respect to a, b and c) such that the ethylene oxide constituent comprises from about 65% to about 75% by weight, of the copolymer molecule and the copolymer has an average molecular weight of from about 2,000 to about 15,000 with the copolymer being present in the tooth whitening composition in such concentration that the composition is a gel at room temperature (about 23 °C).

One block copolymer for use herein is available commercially from BASF and designated PLURAFLO L1220, which has an average molecular weight of 9,800. The hydrophilic poly (ethylene oxide) block averages 65% by weight of the polymer.

At least one additional adhesion enhancing agent in addition to the fumed silica and petrolatum that may be employed in compositions of various embodiments disclosed herein may be present in an amount ranging from about 1 % to about 80% by weight, relative to the total weight of the tooth whitening composition, such as, for example, ranging from about 1.5% to about 75%) by weight.

### Whitening Agents

The tooth whitening compositions disclosed herein comprise at least one whitening agent as a main active ingredient. The at least one whitening agent is a cross-linked polyvinylpyrrolidone-H₂O₂ complex (hereinafter "PVP-H₂O₂"). Polyvinylpyrrolidone is also known as poly-N-vinyl-poly-2-pyrrolidone and commonly abbreviated to "PVP". PVP generally refers to a polymer containing vinylpyrrolidone (also referred to as N-vinylpyrrolidone, N-vinyl-2-pyrrolidione and N-vinyl-2-pyrrolidinone) as a monomeric unit. The monomeric unit consists of a polar imide group, four non-polar methylene groups and a non-polar methane group.

Both linear and cross-linked complexes of PVP-H₂O₂ are known in the art, and PVP-H₂O₂ is considered to be stable in an anhydrous environment. Upon exposure to highly aqueous environments, such as in the oral cavity, the PVP-H₂O₂ dissociates into individual species (PVP polymer and H₂O₂). In one embodiment, the cross-linked PVP-H2O₂ complex is 80% by weight polyvinylpyrrolidone and 20% by weight H₂O₂.

In certain embodiments, the tooth whitening composition may further comprise at least one agent to enhance release of the peroxide in the oral cavity as a part of the peroxide component whitening agent. POLY-PORE, which is an allyl methacrylate crosspolymer, available from Amcol health & Beauty Solutions, Inc., is an exemplary enhancing agent.

In the embodiments disclosed herein, the at least one whitening agent should not degrade in the tooth whitening composition, but rather should be stable in the presence of the other ingredients, such as, for example, the at least one cationic quaternary ammonium compound CPC. In certain embodiments, the hydrogen peroxide is present in the tooth whitening composition in an amount of at least about 70%, such as at least about 80% or at least about 90%, of its initial amount, by weight based on the total weight of the composition, after a time period of at least about one month at room temperature, for example, at least about two months or at least about three months.

In various embodiments, the at least one whitening agent is present in the tooth whitening composition in an amount ranging from about 0.035% to 17.5%, such as from about 0.1% to about 10%, or from about 0.1% to about 6%, by weight relative to the total weight of the tooth whitening composition.

### Additional Ingredients

As previously described, many other components may further be included in the tooth whitening compositions disclosed herein, and include surfactants, flavoring agents, sweetening agents, desensitizing agents, additional anti-microbial agents, anti-caries agents, anti-calculus agents, anti-inflammatory agents, vitamins, pigments and coloring agents, enzymes, preservatives, and tartar control agents, for example.

In certain embodiments disclosed herein, the tooth whitening composition may further comprise at least one flavoring agent. The at least one flavoring agent, may, for example, be selected from essential oils, as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Of these, the most commonly employed are the oils of peppermint, spearmint and wintergreen. The flavoring agent may be incorporated in the tooth whitening compositions disclosed herein at a concentration ranging from 0.01 % to about 2% by weight, such as about 0.1% to about 0.6% by weight.

In embodiments where the tooth whitening composition is sweetened, at least one sweetening agent may be used as an alternative or as a complement to the at least one flavoring agent. Suitable sweetening agents may include, for example, sodium saccharin, sodium cyclamate, xylitol, perillartien, D-tryptophan, aspartame, dihydrochalcones and the like. The at least one sweetening agent may be present in the tooth whitening composition in an amount ranging from about 0.01% to about 1% by weight, such as about 0.3%.

In addition to the at least one cationic quaternary ammonium compound, the tooth whitening compositions disclosed herein may further comprise at least one additional antimicrobial agent. Exemplary antimicrobial agents may include those typically used in oral care compositions, such as Triclosan, chlorhexidine, copper-, zinc- and stannous salts such as zinc citrate, zinc sulfate, zinc glycinate, sodium zinc citrate and stannous pyrophosphate, sanguinarine extract, metronidazole, bis-guanides, such as chlorhexidine digluconate, hexetidine, octenidine, alexidine; and halogenated bisphenolic compounds, such as 2,2'methylenebis-(4-chloro-6-bromophenol).

Exemplary anti-inflammatory agents may include those typically used in oral care compositions, such as ibuprofen, flurbiprofen, aspirin, indomethacine. Exemplary anti-caries agents may include ingredients such as sodium-, calcium-, magnesium-and stannous fluoride, aminefluorides, disodium monofluorophosphate and sodium trimetaphosphate. Exemplary vitamins may include ingredients such as Vitamin C. Exemplary desensitizing agents may include ingredients such as potassium citrate, potassium chloride, potassium tartrate, potassium bicarbonate, potassium oxalate, potassium nitrate and strontium salts. Exemplary anti-calculus agents may include ingredients such as pyrophosphate salts including the mono, di, tri and tetra alkali metal and ammonium pyrophosphate and tripolyphosphate salts. Exemplary enzymes may include papain and glucoamylase.

Some embodiments provide compositions wherein at least one of the ingredients is a fluoride ion source selected from stannous fluoride, sodium fluoride, potassium fluoride, sodium monofluorophosphate, sodium fluorosilicate, ammonium fluorosilicate, amine fluoride, and ammonium fluoride.

In one embodiment, the tooth whitening compositions disclosed herein may comprise the following general formula:

| **Ingredient** | **Amount (w/w)** |
|---|---|
| Trimethylsiloxysilicate/dimethiconol crosspolymer | 12.0% |
| Dimethicone | QS |
| PVP | 18.0-25.0% |
| Hydrogen peroxide | 0.1-6.0% |
| Sodium saccharin | 0.3% |
| Cetylpyridinium chloride | 0.05-0.20% |
| Petrolatum | 25% |
| Fumed silica | 1.0-2.0% |
| Flavor | 0.6% |
| Total: | 100.0% |

In other embodiments, the tooth whitening compositions disclosed herein may comprise the following general formula:

| **Ingredient** | **Amount (w/w)** |
|---|---|
| Trimethylsiloxysilicate/dimethiconol crosspolymer | 12.0% |
| Dimethicone | QS |
| PVP | 18.0-25.0% |
| Hydrogen peroxide | 0.1-6.0% |
| Sodium saccharin | 0.3% |
| Cetylpyridinium chloride | 0.05-0.20% |
| Polyethylene | 1.5-2.0% |
| Mineral oil | 25.0-35.0% |
| Flavor | 0.6% |
| Total: | 100.0% |

Also disclosed herein in various embodiments are provided tooth whitening kits. Such a tooth whitening kit may, for example, comprise a device for applying a tooth whitening composition, and an anhydrous tooth whitening composition comprising at least one hydrophobic polymer carrier, at least one cationic quaternary ammonium compound such as CPC, and at least one whitening agent, wherein the composition is free of water.

In certain embodiments, the tooth whitening kit may comprise a toothbrush having a detachable handle portion forming a dispenser that contains a tooth whitening composition. The dispenser may, for example, be configured as a dispensing pen having a dispensing orifice or applicator at one end. The dispenser may include a ratchet-type fluid dispensing system for delivering the tooth whitening composition. A user may detach the dispenser from the toothbrush, apply the tooth whitening composition to an oral surface, and remount the dispenser in the toothbrush for storage. In certain embodiments the dispenser is to be used for multiple treatments, and therefore the tooth whitening composition should remain stable, without an undesirable build-up of bacteria or associated odors. In certain embodiments, the tooth whitening composition remains stable at room temperature for a time period of at least about one month, such as at least about two months or at least about three months.

In various embodiments, disclosed herein are methods for reducing microbial growth on the surface of an oral care device which comprise applying a tooth whitening composition to the oral care device, wherein the tooth whitening composition comprises at least one hydrophobic carrier, CPC, and a cross-linked PVP-H₂0₂ complex whitening agent, wherein the tooth whitening composition is free of water. In certain embodiments of the methods for reducing microbial growth on the surface of an oral care device, the oral care device is contacted with saliva due to consumer use. Microbial growth on the surface of an oral care device after use may be reduced for a time period of at least about one month, such as at least about two months or at least about three months.

Further disclosed herein are methods for treating or preventing malodor associated with microbial growth on the surface of an oral care device which comprise applying a tooth whitening composition to the oral care device, wherein the tooth whitening composition comprises at least one hydrophobic carrier, CPC, and a cross-linked PVP-H₂0₂ complex whitening agent, wherein the tooth whitening composition is free of water. In certain embodiments of the methods for treating or preventing malodor, the oral care device is contacted with saliva due to consumer use. In the methods disclosed herein, the malodor on the surface of the oral care device resulting from microbial growth may be treated or prevented for a time period of at least about one month, such as at least about two months or at least about three months.

Also disclosed herein are methods for whitening a surface of a tooth in an oral cavity of a human or other animal subject which comprises (a) applying an anhydrous tooth whitening composition as disclosed herein to the tooth surface to be whitened for a plurality of minutes per day; and (b) repeating step (a) for multiple days to thereby whiten the teeth. Also disclosed herein is a method for whitening a surface of a tooth comprising applying to the surface of the tooth a composition comprising at least one hydrophobic polymer carrier, CPC, and cross-linked PVP-H₂0₂ complex whitening agent, wherein the composition is free of water. Exemplary methods disclosed herein comprise contacting the tooth whitening composition with the surface of the tooth. The contacting may occur for a duration of time sufficient to satisfactorily effect whitening of the teeth. Thus, the contacting occurs for a sufficient period of time to at least partially whiten teeth. This can be a period of time ranging from about 1 minute to about 2 hours or longer. In certain embodiments, the contacting is for a period of time ranging from about 1 minute to about 5 minutes, from about 1 minute to about 45 minutes, from about 5 minutes to about 45 minutes, or from about 5 minutes to about 30 minutes.

Further disclosed herein are methods of making a tooth whitening composition as disclosed herein. The tooth whitening compositions disclosed herein may, in certain embodiments, be prepared by adding and mixing the ingredients of the composition in a suitable vessel, such as a stainless steel tank provided with a mixer. In the preparation of the anhydrous tooth whitening composition, the ingredients may be added to the mixer in the following order: hydrophobic polymer component, such as the silicone based pressure sensitive polymer; cetylpyridinium chloride; peroxide whitening agent; adhesion enhancing agents; and any desired flavoring or sweetener. The cetylpyridinium chloride may be added at any time in the mixing process. The ingredients may then be mixed to form a homogenous dispersion/solution.

The tooth whitening compositions disclosed herein may be prepared in the form of a flowable viscous liquid dispersion, such as a gel, comprising cetylpyridinium chloride and at least one whitening agent and is applied as such to the user's teeth as by painting the teeth with a soft applicator brush. Application by the user leaves a coating of the composition on the teeth. Contact with saliva causes the slow release of H₂O₂ from the hydrophobic material matrix to the applied tooth site from the composition, providing prolonged whitening treatment of the tooth sites.

### EXAMPLES

Four different antimicrobial ingredients were evaluated in gel compositions including: (1) cetylpyridinium chloride (CPC); (2) benzyl alcohol, a known preservative that is soluble in oil and used in toothpaste formulations; (3) ethanol, a known antiseptic that is used to inhibit bacterial growth in mouthwash; and (4) tert-butylhydroquinone (TBHQ), a preservative that may be used in foods, such as food oils. Table 1 below shows the formulation details of the four prototypes prepared.

**Table 1: Prototype formulation details**

| **Ingredient** | **Prototype percentage (w/w)** |
|---|---|
| Trimethylsiloxysilicate/dimethiconol crosspolymer | 12.0% |
| Dimethicone | QS |
| PVP | 18.0-25.0% |
| Hydrogen peroxide | 0.1-6.0% |
| Sodium saccharin | 0.3% |
| Polyethylene | 1.5-2.0% |
| Mineral oil | 25.0-35.0% |
| Flavor | 0.6% |
| Anti-microbial ingredient (CPC, benzyl alcohol, ethanol, or TBHQ) | ≤ 10.0% |
| Total | 100.0% |

The prototypes were challenged against several acceptance criteria, as will be discussed in the sections below. First, the prototypes were aged according to International Conference on Harmonisation (ICH) conditions to determine if the ingredients compromised the physical/shelf stability of the prototypes prepared or the chemical stability of hydrogen peroxide. Second, a laboratory method was developed to simulate bacterial malodor development on the external surfaces of an oral care applicator. Third, a laboratory method was developed to characterize the antimicrobial preservative efficacy of the prototype formula options.

### Part A - Stability Testing and Results

Physical and chemical stability was evaluated for up to three months using guidelines from the ICH accelerated aging conditions (i.e., 40 °C and 75% relative humidity). Table 2 below details the results of the stability testing for each of the four prototypes prepared, as well as a control formulation that was prepared containing no anti-microbial ingredient. After aging, the percentage of hydrogen peroxide was measured to determine whether the hydrogen peroxide had degraded or remained stable in the composition. A hydrogen peroxide recovery of greater than or equal to 70% was determined to be a stable solution. The compositions were visually inspected for physical attributes to determine whether they conformed to stability standards. A composition was considered to pass if there was no discernable change in physical characteristics as compared to the initial (i.e., unaged) observation. A composition was considered to fail if there was a consumer noticeable change in physical characteristics such as liquid/solid phase separation, aeration, discoloration, changes in viscosity, and/or changes in adhesive properties.

**Table 2: Stability Test Results**

| **Prototype** | **Physical attributes** | **Hydrogen peroxide recovery** |
|---|---|---|
| Unpreserved control | Pass/conforms to standards | Pass (≥70% H₂O₂ recovered) |
| CPC prototype | Pass/conforms to standards | Pass (≥70% H₂O₂ recovered) |
| Benzyl alcohol prototype | Fail/does not conform | Pass (≥70% H₂O₂ recovered) |
| TBHQ prototype | Pass/conforms to standards | Pass (≥70% H₂O₂ recovered) |
| Ethanol prototype | Fail/does not conform | Pass (≥70% H₂O₂ recovered) |

### Part B - Odor Evaluation (In Vitro)

The four prototypes and the control were tested to screen the various anti-microbial ingredients for malodor. An odor mixture was created by mixing whole saliva from two to three donors together with full strength fluid thioglycollate (FTG) media. The four prototypes and the control were then subjected to a first level of challenge. Every formula that passed the first level challenge went on to a second level of challenge. If the formula failed the first level challenge, it was eliminated from the study.

For the first level of challenge, to simulate consumer use, an applicator pen was first primed to dispense the prototype formula being tested. The formula was dispensed by two to three pen clicks and smeared around the tip of the applicator. The tip was then treated with 40 µl of the odor mixture. The toothbrush cavity was also treated with the odor mixture, and the treated pen was then placed back in the toothbrush cavity. The first level challenge consisted of twice-a-day treatment with the odor mixture with an incubation period of 4 to 5 hours at 37 °C in between treatments. After the 4 to 5 hour incubation period, the pens were allowed to return to room temperature and then tested for odor by an organoleptic expert, and a pass/fail rating was assigned for each formula depending on whether or not the organoleptic expert was able to discern the malodor associated with microbial growth on the pen. If the pen smelled of microbial growth, the pen failed the first level challenge. The results of the Level 1 challenge are detailed below in Table 3.

**Table 3: Odor Evaluation, Level 1 Challenge**

| **Prototype** | **Results** |
|---|---|
| Unpreserved control | Fail |
| CPC prototype | Pass |
| Benzyl alcohol prototype | Borderline pass |
| TBHQ prototype | Fail |
| Ethanol prototype | Pass |

The pens were then subjected to a second level challenge by repeating the first level challenge treatment, followed by another odor evaluation by an organoleptic expert. The results are detailed below in Table 4.

**Table 4: Odor Evaluation**

| **Prototype** | **Results** |
|---|---|
| Unpreserved control | Fail |
| CPC prototype | Pass |
| Benzyl alcohol prototype | Fail |
| TBHQ prototype | Fail |
| Ethanol prototype | Pass |

### Fart C - Antimicrobial Efficacy Evaluation (In Vitro)

Bacterial challenge organisms were grown in an artificial saliva medium and inoculated with the control formulation and the two prototype formulations that passed the stability study (i.e., the CPC prototype and the TBHQ prototype) in order to test the formulations' ability to withstand microbial insult. Organism recoveries were performed at set time points, and the log reduction of bacteria was measured. Then the area under the curve (AUC) was calculated based on the reduction of organisms over time. Pass/fail criteria were based on a comparison of the AUC for the prototype products versus that of a known standard. Table 5 below details the results of the antimicrobial efficacy study.

**Table 5: Antimicrobial Efficacy**

| **Prototype** | **Results** |
|---|---|
| Unpreserved control | Fail |
| CPC prototype | Pass |
| Benzyl alcohol prototype | Not tested due to stability failure |
| TBHQ prototype | Fail |
| Ethanol prototype | Not tested due to stability failure |

A formula prototype containing 0.1% CPC and a control formulation without CPC were tested in a three-month usage study with consumers to confirm that the previously identified malodor and microbial build-up concerns were addressed by the addition of CPC. The CPC prototype showed improvements in the following areas: (1) reduced malodor complaints compared to unpreserved prototype previously tested; (2) reduced malodor in used samples when judged by an organoleptic expert; (3) reduced counts of aerobic bacteria; and (4) reduced counts of yeast and mold. For the control formulation without CPC, 3% of the consumer subjects discontinued use due to malodor. For the formula prototype containing 0.1% CPC, less than 1% of the consumer subjects discontinued use due to malodor.

## Claims

1. A tooth whitening composition comprising:
at least one hydrophobic polymer carrier;
cetylpyridinium chloride; and
a cross-linked PVP-H₂O₂ complex whitening agent;
wherein the tooth whitening composition is anhydrous.

2. The tooth whitening composition according to claim 1, wherein the hydrophobic polymer carrier is a silicone pressure sensitive adhesive.

3. The tooth whitening composition according to claims 1 or 2, wherein the cross-linked PVP-H₂O₂ complex comprises from 0.1% to 6.0% by weight hydrogen peroxide, relative to the total weight of the composition.

4. The tooth whitening composition according to any of the preceding claims, wherein the cetylpyridinium chloride is present in the composition in an amount ranging from 0.05% to 0.20% by weight relative to the total weight of the composition.

5. The tooth whitening composition according to any of the preceding claims, wherein the cetylpyridinium chloride is present in the tooth whitening composition in an amount of about 0.1% by weight relative to the total weight of the composition.

6. The tooth whitening composition according to any of the preceding claims, further comprising at least two adhesion enhancing agents.

7. The tooth whitening composition according to claim 6, wherein the at least two adhesion enhancing agents are fumed silica and petrolatum.

8. The tooth whitening composition according to any of the preceding claims, wherein the composition is free of polyethylene.

9. The tooth whitening composition according to any of the preceding claims, wherein the composition is in the form of a gel.

10. A method for reducing microbial growth on the surface of an oral care device comprising:
contacting the oral care device with saliva and a tooth whitening composition,
wherein the tooth whitening composition comprises at least one hydrophobic polymer
carrier, cetylpyridinium chloride, and a cross-linked PVP-H₂O₂ complex whitening agent, and
wherein the tooth whitening composition is anhydrous.

11. The method according to claim 10, wherein a cross-linked PVP-H₂O₂ complex comprises from 0.1% to 6.0% by weight hydrogen peroxide, relative to the total weight of the composition, and/or wherein after the contacting step, the oral care device is free of malodor caused by bacterial growth for a time period of at least three months.

12. The method according to claim 10 or 11, wherein the cetylpyridinium chloride is present in the composition in an amount ranging from 0.05% to 0.20% by weight relative to the total weight of the composition, optionally wherein the cetylpyridinium chloride is present in the composition in an amount of 0.1% by weight relative to the total weight of the composition.

13. The method according to any one of claims 10 to 12, wherein the tooth whitening composition further comprises at least two adhesion enhancing agents, optionally wherein the at least two adhesion enhancing agents are fumed silica and petrolatum.

14. The method according to any one of claims 10 to 13, wherein the tooth whitening composition is free of polyethylene.

15. The method according to any one of claims 10 to 14, wherein the tooth whitening composition is in the form of a gel.

## Patentansprüche

1. Zahnaufhellungszusammensetzung, umfassend:
mindestens einen hydrophoben Polymerträger;
Cetylpyridiniumchlorid; und
ein Aufhellungsmittel aus vernetztem PVP-H₂O₂-Komplex;
wobei die Zahnaufhellungszusammensetzung wasserfrei ist.

2. Zahnaufhellungszusammensetzung gemäß Anspruch 1, wobei der hydrophobe Polymerträger ein druckempfindlicher Silikonklebstoff ist.

3. Zahnaufhellungszusammensetzung gemäß Ansprüchen 1 oder 2, wobei der vernetzte PVP-H₂O₂-Komplex von 0,1 Gewichts-% bis 6,0 Gewichts-% Wasserstoffperoxid, bezogen auf das Gesamtgewicht der Zusammensetzung, umfasst.

4. Zahnaufhellungszusammensetzung gemäß einem beliebigen der vorhergehenden Ansprüche, wobei das Cetylpyridiniumchlorid in der Zusammensetzung in einer Menge im Bereich von 0,05 Gewichts-% bis 0,20 Gewichts-%, bezogen auf das Gesamtgewicht der Zusammensetzung, vorliegt.

5. Zahnaufhellungszusammensetzung gemäß einem beliebigen der vorhergehenden Ansprüche, wobei das Cetylpyridiniumchlorid in der Zahnaufhellungszusammensetzung in einer Menge von etwa 0,1 Gewichts-%, bezogen auf das Gesamtgewicht der Zusammensetzung, vorliegt.

6. Zahnaufhellungszusammensetzung gemäß einem beliebigen der vorhergehenden Ansprüche, weiterhin mindestens zwei haftungsverstärkende Mittel umfassend.

7. Zahnaufhellungszusammensetzung gemäß Anspruch 6, wobei die mindestens zwei haftungsverstärkenden Mittel pyrogenes Siliziumdioxid und Petrolatum sind.

8. Zahnaufhellungszusammensetzung gemäß einem beliebigen der vorhergehenden Ansprüche, wobei die Zusammensetzung frei von Polyethylen ist.

9. Zahnaufhellungszusammensetzung gemäß einem beliebigen der vorhergehenden Ansprüche, wobei die Zusammensetzung in der Form eines Gels vorliegt.

10. Verfahren zur Verringerung des mikrobiellen Wachstums an der Oberfläche einer Mundpflegevorrichtung, umfassend:
Inkontaktbringen der Mundpflegevorrichtung mit Speichel und einer Zahnaufhellungszusa mmensetzung,
wobei die Zahnaufhellungszusammensetzung mindestens einen hydrophoben Polymerträger, Cetylpyridiniumchlorid und ein Aufhellungsmittel aus vernetztem PVP-H₂O₂-Komplex umfasst, und
wobei die Zahnaufhellungszusammensetzung wasserfrei ist.

11. Verfahren gemäß Anspruch 10, wobei ein vernetzter PVP-H₂O₂-Komplex von 0,1 Gewichts-% bis 6,0 Gewichts-% Wasserstoffperoxid, bezogen auf das Gesamtgewicht der Zusammensetzung, umfasst, und/oder wobei die Mundpflegevorrichtung nach dem Schritt des Inkontaktbringens für einen Zeitraum von mindestens drei Monaten frei von durch bakterielles Wachstum verursachtem schlechten Geruch ist.

12. Verfahren gemäß Anspruch 10 oder 11, wobei das Cetylpyridiniumchlorid in der Zusammensetzung in einer Menge im Bereich von 0,05 Gewichts-% bis 0,20 Gewichts-%, bezogen auf das Gesamtgewicht der Zusammensetzung, vorliegt, wobei das Cetylpyridiniumchlorid in der Zusammensetzung gegebenenfalls in einer Menge von 0,1 Gewichts-%, bezogen auf das Gesamtgewicht der Zusammensetzung, vorliegt.

13. Verfahren gemäß einem beliebigen der Ansprüche 10 bis 12, wobei die Zahnaufhellungszusammensetzung weiterhin mindestens zwei haftungsverstärkende Mittel umfasst, wobei die mindestens zwei haftungsverstärkenden Mittel gegebenenfalls pyrogenes Siliziumdioxid und Petrolatum sind.

14. Verfahren gemäß einem beliebigen der Ansprüche 10 bis 13, wobei die Zahnaufhellungszusammensetzung frei von Polyethylen ist.

15. Verfahren gemäß einem beliebigen der Ansprüche 10 bis 14, wobei die Zahnaufhellungszusammensetzung in der Form eines Gels vorliegt.

## Revendications

1. Composition de blanchiment des dents comprenant :
au moins un support polymère hydrophobe ;
du chlorure de cétylpyridinium ; et
un agent de blanchiment complexe PVP-H₂O₂ réticulé ;
dans laquelle la composition de blanchiment des dents est anhydre.

2. Composition de blanchiment des dents selon la revendication 1, dans laquelle le support polymère hydrophobe est un adhésif silicone sensible à la pression.

3. Composition de blanchiment des dents selon la revendication 1 ou 2, dans laquelle le complexe PVP-H₂O₂ réticulé comprend de 0,1 % à 6,0 % en poids de peroxyde d'hydrogène, par rapport au poids total de la composition.

4. Composition de blanchiment des dents selon l'une quelconque des revendications précédentes, dans laquelle le chlorure de cétylpyridinium est présent dans la composition en une quantité allant de 0,05 % à 0,20 % en poids par rapport au poids total de la composition.

5. Composition de blanchiment des dents selon l'une quelconque des revendications précédentes, dans laquelle le chlorure de cétylpyridinium est présent dans la composition de blanchiment des dents en une quantité d'environ 0,1 % en poids par rapport au poids total de la composition.

6. Composition de blanchiment des dents selon l'une quelconque des revendications précédentes, comprenant en outre au moins deux agents améliorant l'adhérence.

7. Composition de blanchiment des dents selon la revendication 6, dans laquelle les au moins deux agents améliorant l'adhérence sont la silice sublimée et la vaseline.

8. Composition de blanchiment des dents selon l'une quelconque des revendications précédentes, dans laquelle la composition est exempte de polyéthylène.

9. Composition de blanchiment des dents selon l'une quelconque des revendications précédentes, dans laquelle la composition est sous la forme d'un gel.

10. Procédé pour réduire la croissance microbienne à la surface d'un dispositif de soins bucco-dentaires comprenant :
la mise en contact du dispositif de soins bucco-dentaires avec de la salive et une composition de blanchiment des dents,
dans lequel la composition de blanchiment des dents comprend au moins un support polymère hydrophobe, du chlorure de cétylpyridinium et un agent de blanchiment complexe PVP-H₂O₂ réticulé, et
dans lequel la composition de blanchiment des dents est anhydre.

11. Procédé selon la revendication 10, dans lequel un complexe PVP-H₂O₂ réticulé comprend de 0,1 % à 6,0 % en poids de peroxyde d'hydrogène, par rapport au poids total de la composition, et/ou dans lequel après l'étape de mise en contact, le dispositif de soins bucco-dentaires est exempt de mauvaises odeurs causées par la croissance bactérienne pendant une période d'au moins trois mois.

12. Procédé selon la revendication 10 ou 11, dans lequel le chlorure de cétylpyridinium est présent dans la composition en une quantité allant de 0,05 % à 0,20 % en poids par rapport au poids total de la composition, éventuellement dans lequel le chlorure de cétylpyridinium est présent dans la composition en une quantité de 0,1 % en poids par rapport au poids total de la composition.

13. Procédé selon l'une quelconque des revendications 10 à 12, dans lequel la composition de blanchiment des dents comprend en outre au moins deux agents améliorant l'adhérence, éventuellement dans lequel les au moins deux agents améliorant l'adhérence sont la silice sublimée et la vaseline.

14. Procédé selon l'une quelconque des revendications 10 à 13, dans lequel la composition de blanchiment des dents est exempte de polyéthylène.

15. Procédé selon l'une quelconque des revendications 10 à 14, dans lequel la composition de blanchiment des dents se présente sous la forme d'un gel.
